# EUROPEAN PATENT APPLICATION

(11) **EP 1 621 880 A1**
(43) Date of publication of application: **01.02.2006**
(21) Application number: 04103684.9
(22) Date of filing: 30.07.2004
(51) Int. Cl.: G01N 30/02, G01N 37/00

(54) **Method for modulating the "key-lock" binding-affinity of molecules**

(71) Applicant: The European Community, represented by the European Commission, 1049 Brussels (BE)
(72) Inventor: Stroka, Joerg, 47137 Duisburg (DE); Rossi, François, 21024 BI Andronno (IT); Anklam, Elke, 2740 Retie (BE)
(74) Representative: Kihn, Pierre Emile Joseph

(57) **Abstract**

The invention concerns a method for modulating the "key-lock" binding-affinity of molecules, wherein the molecules are submitted to an electrical field of between 1 kV/m and 10 MV/m and having an oscillating frequency of between 0.001 MHz and 20 GHz.

## Description

### Introduction

The present invention relates to a method for modulating the "key-lock" binding-affinity of molecules.

### Background of the invention

A variety of analytical techniques are used to characterize interactions between molecules, particularly in the context of assays directed to the detection and interaction of biomolecules. For example, antibody-antigen interactions are fundamental importance in many fields, including biology, immunology and pharmacology. In this context, many analytical techniques involve binding a "ligand" (such as an antibody) to a solid support, followed by contacting the ligand with an "analyte" (such as an antigen) Following contact of the ligand and analyte, some characteristic is measured which is indicative of the interaction, such as the ability to of the ligand to bind the analyte. After measurement of the interaction, the ligand-analyte pair must be disrupted in order to "regenerate" free ligand for further analytical measurement in case of Biosensors. In case of purification applications, the analyte is released from the ligand after purification from other substances, for which the ligand is not specific.

A number of techniques have been employed to regenerate surface-bound ligands. Most commonly, regeneration involves a series of trial and error attempts to remove the analyte from the ligand, while minimizing loss of ligand from the solid support. Care must also be taken not to use a regeneration solution (or a regeneration process), which is too aggressive in order to avoid partial or complete loss of ligand activity. Furthermore, regeneration must not influence the ligand with regard to subsequent measurements; otherwise results from assay to assay will not be truly comparable. These problems may be avoided by simply discarding the solid support after each assay. However, this is undesirable since generation of the solid support having bound ligand can be both costly and time consuming, and very often the researchers has only limited quantities of the ligand and/or solid support. Accordingly, improved techniques for regenerating such surfaces are desired.

### Object of the invention

The object of the present invention is to provide improved techniques for regenerating such surfaces as desired

### General description of the invention

In order to overcome the abovementioned problems, the present invention discloses a method for modulating the "key-lock" binding-affinity of molecules, wherein the molecules are submitted to an electrical field of between 1 kV/m and 10 MV/m and having an oscillating frequency of between 0.001 MHz and 20 GHz.

The "key-lock" binding-affinity as understood in the context of the present invention is a force that is characterised by the fact that two matching molecules develop an interaction that appears as a mutual attraction between them.

Consequently, the method of the invention allows influencing the equilibrium constant K of a ligand-analyte pair. On the one hand, applying the above-described electrical field may be used to decrease the equilibrium constant of a ligand-analyte pair so that the regeneration of the surfaces is enhanced. On the other hand, the above-described electrical field may be used to increase the equilibrium constant of a ligand-analyte pair so that a stronger attraction between them is achieved - i.ethe analyte is bound stronger a specific surface.

A definition and examples of the ligand-analyte pair is given in US 6,503,760.

According to another aspect of the present invention, there is disclosed a method for discriminating between a specific binding and an unspecific binding of a molecule to a binding site, wherein the molecule and the binding site are submitted to an electrical field of between 1 kV/m and 10 MV/m and having an oscillating frequency of between 0.001 MHz and 20 GHz. As a result the field can be used to study the changes of the binding affinity, which can be used for analytical purposes in various biological and chemical applications (such as surface plasmon resonance).

According to a first embodiment, the electrical field is at least 10kV/m, preferably at least 100 kV/m and most preferably at least 500 kV/m.

The electrical field is advantageously 5 MV/m max., preferably 2 MV/m max., and most preferably 900 kV/m max.

According to a preferred embodiment, the oscillating frequency of the electrical field is at least 0.01 MHz preferably at least 0.1 MHz and most preferably at least 1 MHz.

According to a preferred embodiment, the oscillating frequency of the electrical field is 10 GHz max., preferably 1GHz max. and most preferably 2 MHz max.

### Detailed description with respect to the figures

The present invention will be more apparent from the following description of a not limiting embodiment with reference to the attached drawings, wherein
Fig. 1 shows the schematic set-up of the experiment;
Fig. 2 represents a snapshot of the oscillator and the shielding box with Teflon housing containing the filed plates;
Fig. 3 depicts a comparison of aflatoxins on the upper IAC (IAC1)
Fig. 4 is a comparison of the binding of aflatoxins as collected on the lower IAC (IAC2) from the elution of the upper IAC (IAC1) during the experiment.
Fig. 5 represents the signals for aflatoxin B2 in the subsequent eluates (1,5 mL each) when eluted under alternating conditions with the electrical field "ON" and "OFF".
Fig. 6 shows the signals for aflatoxin G2 in the subsequent eluates (1,5 mL each) when eluted under alternating conditions with the electrical field "ON" and "OFF".

The effect of an oscillating electrical field, when applied to immobilized antibodies (immunoaffinity column) is now described in more detail.

The tested immunoaffinity column (IAC) was specific to aflatoxins. It could be shown that an oscillating electrical field in the range of 0.1 MHz had a significant effect on the interaction of the antibody-antigen binding (AAB). The effect observed, showed that IACs have a lower affinity to the antigen when applying an electrical field.

*Since mycotoxins are* a *potential hazard to humans and the fact that legislative limits have been proposed in more than 77 countries worldwide, the regular monitoring* of *these substances is a constant challenge for the analyst. About* a decade ago, the availability of commercial immunoaffinity columns (IAC) for the purification and concentration of these toxins from food and feed matrices has significantly improved analytical procedures. Generally the mycotoxins are applied on the IAC in form of diluted extracts, containing either up to 10% of methanol or up 2-3% of acetone or acetonitrile as described by Scott, P.M. & Trucksess, M.W. (1997). Application of immunoaffinity columns to mycotoxin analysis, Journal of the AOAC International, 80: 941-919.

After purification, the mycotoxins are normally eluted with neat methanol or acetonitrile by breaking the antibody-antigen binding (AAB), so that they are available for further analytical procedures. Such an elution with a neat organic solvent can lead to the destruction of the antibody and therefore other ways to detach the antigen from the antibody are of interest.

In electrophoresis, it is known for a long time to apply a static electrical fields to force the migration of charged substances through a gel. This procedure allows to separate substances, or substance clusters with different mass to charge ratios (m/z). It has also been shown that antibodies can migrate in such field without any harm to their ability to bind antigens. However, the field forces applied were comparable small with normally less than a 2 kV/m.

However, since the interaction between antibody and antigen is of complex nature, involving ionic, van-der-Waals and hydrogen-bond, the application of very strong oscillating fields (>10 MV/m) on the effect to modulate the AAB was tested. Several different and commercially available IAC, that are either specific to the group of aflatoxins (aflatoxin B₁, B₂, G₁ and G₂), to deoxynivalenol (DON), to fumonsins (FB₁ and FB₂) or to ochratoxin A (OTA), are commercially available. Aflatoxins were tested independently in oscillating electrical fields to study the effect on the AAB.

The aflatoxins B₁, B₂, G₁ and G₂ all have similar structures and are non-ionic substances (not charged in aqueous solutions) and it is known that the used antibodies have a sufficient affinity to all of these four aflatoxins, as they are used for the simultaneous purification of all four substances. Therefore mixtures as well as solution containing single aflatoxins were tested.

When an electrical field is applied on the antibody-antigen complex (AAC) - immobilised antibody and 'free' antigen (in solution) -, the electrical force induced, will interact with the charged regions of the antigens, other charges molecules in solution (e.g. buffer salts) and the AAC.

### Materials and Methods: Oscillator and IAC support:

The oscillator used was constructed from a modified TV fly-back transformer with solid-state drivers.

Due to the fact that a simple set-up only allowed producing frequencies around 0.1 MHz, the oscillator was operated at the given fixed frequency. Voltage and frequency was measured with a 100,1 MΩ cascade divider (100×1MΩ and 1X100kΩ). This cascade served as 1/1000 voltage divider and allowed signal measurement with a HAMEG HM 312 oscilloscope. The resulting voltage was 10 kV (peak-to-peak) at around 97 kHz.

The resulting high-voltage (HV) was applied on two 10x10 cm with Teflon housed (isolated) aluminium plates and the tested IAC mounted between these plates. The distance of the plates was 1.5 cm and the dielectricum was air. These plates functioned as a capacitor, in which the electrical field was induced. This resulted in a field-intensity of (10.000 x 100 / 1.5) = 667 kV/m.

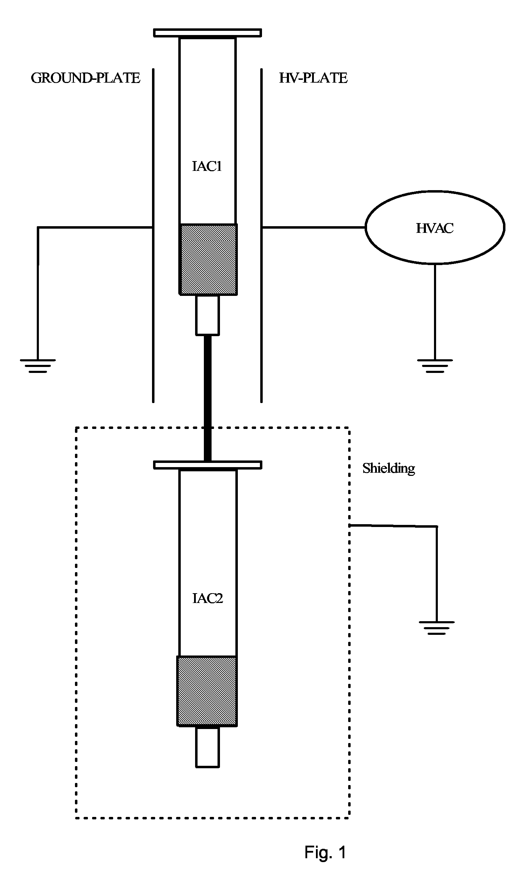

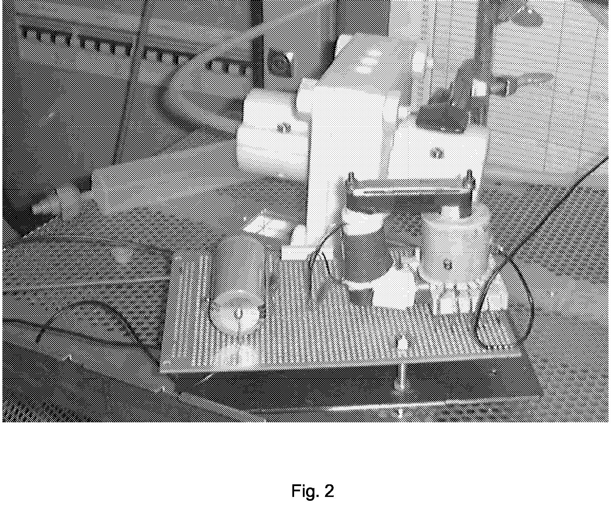

Below this capacitor an aluminium cover was mounted and connected electrically with one of the plates that was grounded. This allowed shielding any other material that was located below the capacitor (Figures 1 & 2). As a matter of fact this construction allowed exposing the upper IAC to the field, while the lower IAC was not affected.

For experiments the upper IAC was first mounted alone without field, loaded beyond its maximum capacity of 100 ng (total aflatoxins) with 10 mL of a mix of aflatoxins B₁, B₂, G₁ and G₂ (concentration of the standard approx. 50 ng/mL total aflatoxins) and then washed with approx. 10 mL of water.

In a second stage after the upper IAC has been washed the lower IAC was connected and the upper IAC (once with and once without field in two different series) further washed flushed with water and the eluate was collected (actual experiment). Both sets of IACs were then eluted with neat methanol and the extract measured by HPLC.

In a second set-up of experiment with only one IAC (previously upper IAC) the IAC was loaded again beyond maximum capacity to achieve max load, shortly washed with approx. 3 mL of water and then flushed with water in portions of 1.5 mL. These portions were collected in small vials, while the HV source (electrical field) was switched on and off alternating. The resulting eluates were analysed for aflatoxins again by HPLC.

### HPLC analysis:

The HPLC system and procedures used were the same as been described in the article of Stroka J, Anklam E, Joerissen U, Gilbert J (2000) Immunoaffinity cleanup with liquid chromatography using post-column bromination for determination of aflatoxins in peanut butter, pistachio paste, fig paste, and paprika powder: collaborative study, Journal of the AOAC International 83: 320-340 [2].

### Results:

Generally the binding of aflatoxins with the antibody is characterised by a constant, that is characteristic for the strength of the AAB. As a result, a minor amount of aflatoxins will also be detached from the antibody during loading. As can be seen, applying an oscillating electrical field can modify the value of this constant.

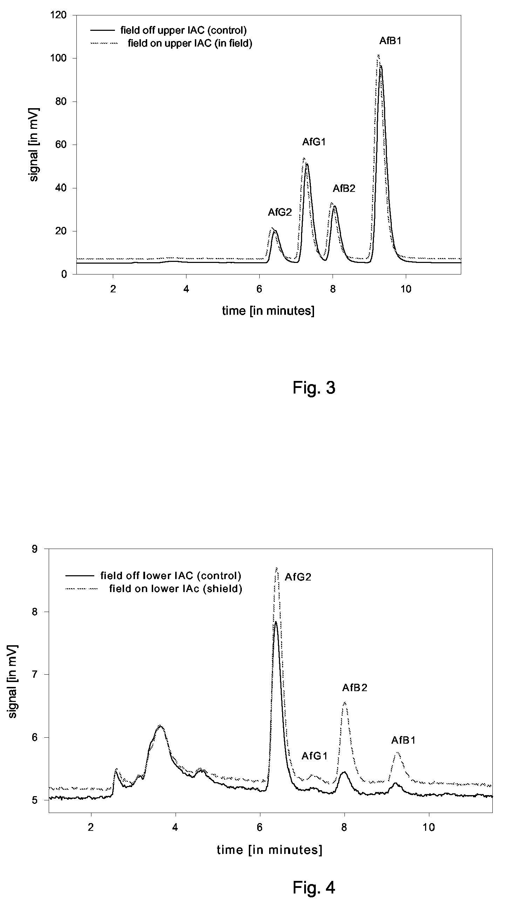

During the experiment the applied washing solution first passed the upper and subsequently the lower IAC. Thus in the lower IAC all the aflatoxins that will by-pass the upper one will be collected. As can be seen the effect of the applied field was not apparent from the analysis of the upper column, but significant observable in the lower IAC. This can be seen in chromatograms of Figure 3 & 4.

The chromatograms show that this interaction is in the case of aflatoxin IACs different for each toxin. Such an applications is of interest wherever the mechanism of 'key-lock principles' is used (e.g. immuno-chemistry or molecular imprinted polymers, so called "plastibodies").

So far the alteration of AAB in immuno-chemistry applications required the application of organic solvents or other chemical substances. This might not effect only the AAB, but also harm the antigen, in case of proteins. The present experiments show that electrical fields do the same, while the observed effect is reversal for the here performed experiments.

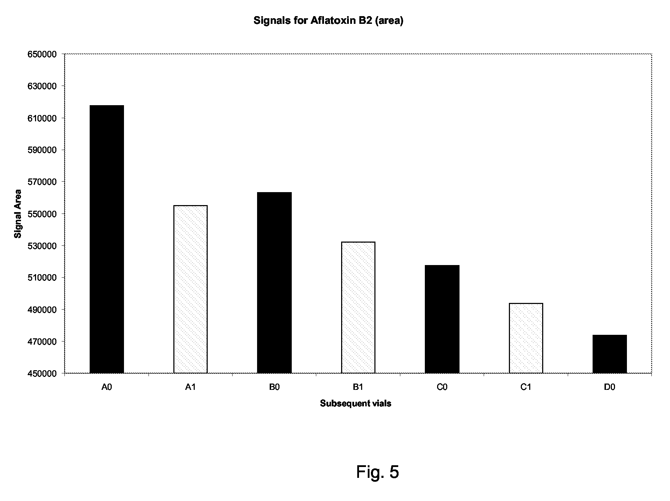

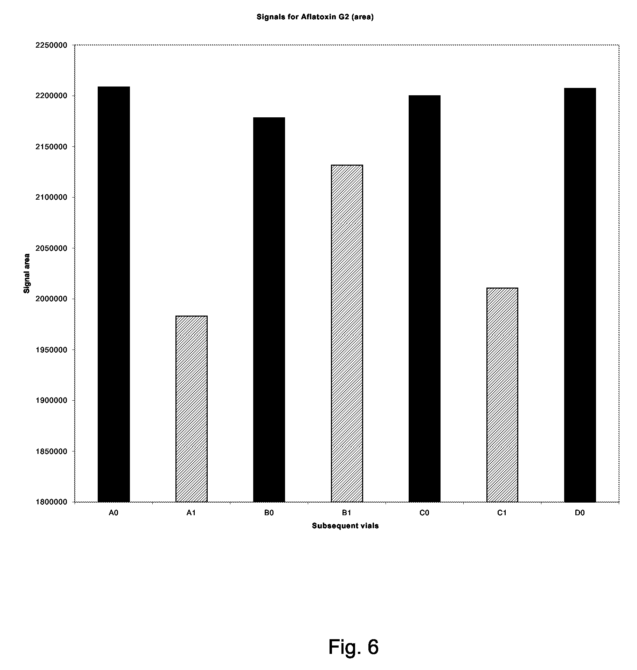

In the second type of experiment (Figures 5 & 6), - alternating applied field - this effect can be nicely underpinned when plotting the obtained signals from a series of eluates (field: OFF-ON-OFF-ON-OFF ).

### Conclusion:

The here-described principle has the potential to interfere with such "key-lock" bound complexes. The field of interest vary from methodologies that involve immunochemical procedures such as enzyme linked immuno-sorbant asseys (ELISA), surface plasmon resonance (SPR) to fluorescence polarisation (FP) or even other techniques, since these technologies directly depend on the binding constant of the antigen-antibody complex. Therefore, the reversible manipulation (switch ON-OFF) of this constant can lead to further features in the identification of antigen and nonspecific binding mechanisms. This is due to the fact that the AAB constant can be used to distinguish between specific binding and non-specific binding.

Furthermore, other interactions between molecules for which a key-lock principle is known (e.g. molecular imprinted polymers (MIP), so called "artificial antibodies" or "plastibodies") with their template counterpart as well as other techniques involving such interaction principles are likely.

The method involves electrical fields; these fields are applied to a medium containing substances that interact with specific binding characteristics between each other. The field is applied around the medium. The field is variable for different analyte ligand systems.

The oscillation of the field may be of different shapes such as sinus, square (pulsed) or of any other shape (e.g. random noise).

The 'on-rate' and 'off-rate' are specific and characteristic constants that specify the affinity of antibodies to their antigen. As a matter of fact the 'on-rate' and 'off-rate' allows to distinguish between 'specific binding' (thus between the antibody and the analyte) and 'unspecific binding' (thus any interference). The modulation of this parameters therefore can find applications in the following fields:
- Interaction between antigen and antibody in products that use antibodies for purification purposes (e.g. immunoaffinity chromatography).
- Interaction between substances from other structures than antibodies (e.g. from molecular imprinted polymers - so called MIPS) for clean-up purposes, similar to immunoaffinity chromatography.
- Application in biochips (biosensors) that use one of the above mentioned principles. Most biosensors use antibodies for measurements.

The force of the electrical field that has to be employed may depend on the dielectric constant of the medium in which the interaction takes place. In the experiments performed in relation to the present invention, the medium was distilled water. The voltage was approx. 10 kV/cm = 1 MV/m.

Additives (whether they are polar, ionic or not-ionic) such as salts, zwitterionic substances, tweens® or spans®, polymers such as (polyvinylpyrollidone, polye-htylene), may be used to aid or amplify the effect.

## Claims

1. A method for modulating the "key-lock" binding-affinity of molecules, wherein the molecules are submitted to an electrical field of between 1 kV/m and 10 MV/m and having an oscillating frequency of between 0.001 MHz and 20 GHz.

2. A method for discriminating between a specific binding and an unspecific binding of a molecule to a binding site, wherein the molecule and the binding site are submitted to an electrical field of between 1 kV/m and 10MV/m and having an oscillating frequency of between 0.001 MHz and 20 GHz.

3. The method according to claim 1 or 2, wherein the electrical field is at least 10kV/m, preferably 100 kV/M and most preferably 500 kV/m.

4. The method according to claim 1 or 2, wherein the electrical field is 5 MV/m max., preferably 2 MV/m max., and most preferably 900 kV/m max.

5. The method according to any of the preceding claims, wherein the oscillating frequency of the electrical field is at least 0.01 MHz preferably at least 0.1 MHz and most preferably at least 1 MHz.

6. The method according to any of the preceding claims, wherein the oscillating frequency of the electrical field is 10 GHz MHz max., preferably 1 GHz max. and most preferably 2 MHz max.
